# FASCICULE DE BREVET EUROPEEN

(11) **EP 1 648 292 B1**
(45) Date de publication et mention de la délivrance du brevet: **26.05.2010**
(21) Numéro de dépôt: 04767807.3
(22) Date de dépôt: 28.07.2004
(51) Int. Cl.: A61B 5/00, H04N 5/14

(54) **APPAREIL DE DETECTION ET DE CARACTERISATION DES TISSUS BIOLOGIQUES**
VORRICHTUNG ZUM NACHWEIS UND ZUR CHARAKTERISIERUNG VON BIOLOGISCHEN GEWEBEN
DEVICE FOR THE DETECTION AND CHARACTERIZATION OF BIOLOGICAL TISSUE

(30) Priorité: 28.07.2003 FR 0309256
(43) Date de publication de la demande: 26.04.2006
(73) Titulaire: Sopro (Societe Anonyme), 13705 La Ciotat Cedex (FR)
(72) Inventeur: MAZUIR, Alain, 83470 MAXIMIN (FR); DIERAS, Francis, F-33000 Bordeaux (FR)
(74) Mandataire: Domange, Maxime
(86) Numéro de dépôt international: PCT/FR2004/002026
(87) Numéro de publication internationale: WO 2005/011486

(56) Documents cités:
- FR-A- 2 825 260
- US-B1- 6 295 322
- US-B1- 6 593 967

## Description

La présente invention concerne un procédé et un dispositif de détection, de localisation, et de caractérisation des différences de densité, de structure ou de composition chimique d'un tissu biologique.

On a proposé, dans l'état antérieur de la technique, divers procédés de détection, ou de mise en évidence, de différences tissulaires, d'origine physiologique, ou histologique, pathologiques ou non, utilisant l'auto-fluorescence des tissus contenant des chromophores endogènes ou la fluorescence provoquée par des colorants administrés ou chromophores exogènes.

On a pu ainsi réaliser une cartographie en temps réel de la fluorescence des tissus vivants, basée sur le principe suivant lequel la teneur en chromophores est différente selon que la zone observée est saine ou lésée.

Une telle méthode a ainsi été utilisée pour l'observation directe des lésions carieuses sur des tissus durs tels que l'émail des dents, ou sur des tissus mous tels que la peau ou la muqueuse buccale, ou, par voie endoscopique, sur les muqueuses endo-cavitaires thoraciques ou abdominales.

On a également proposé divers procédés de détection et de caractérisation de différences tissulaires, dans lesquels on éclaire des tissus au moyen d'une lumière monochromatique de longueur d'onde déterminée, de façon à amener celle-ci à émettre en retour des radiations par luminescence à une longueur d'onde différente.

Selon ce principe et à titre d'exemple en comparant l'intensité de la luminescence émise par une zone saine d'une dent et une zone cariée de celle-ci, on détermine, par des mesures respectives dans ces deux longueurs d'onde spécifiques, notamment par une opération mathématique, faisant la différence de ces deux intensités, la présence d'une carie ou la mise en évidence d'une différenciation tissulaire ou une altération de surface, en fonction de la valeur obtenue.

Une telle méthode a aussi été utilisée pour la détection de processus inflammatoires du pancréas in vivo sur des modèles animaux sur lesquels on a obtenu une discrimination tissulaire significative entre tissus sains et tissus lésés en comparant les spectres et les rapports d'intensité entre le bleu et le rouge.

Le document US 2001/0049473 décrit une méthode et un système pour détecter et localiser une difference de composition d'un tissu biologique soumis a une illumination continue dans une premiere bande de fréquence capable de déclencher un phénomène de fluorescence, auto- fluorescence ou luminescence du tissu. Pour l'acquisition d'une image de fluorescence, ce document utilise des images stockées acquises avec au moms trois pixels différents de couleurs complémentaires de manière a augmenter la sensibilité de detection de la fluorescence d'une manière connue par l'homme du métier.

Pour atteindre son but, ce document utilise la différence de fluorescence extrinsèque et intrinsèque entre tissu normal et tissu malade. En effet, la fluorescence intrinsèque est moms importante pour les zones malades que pour les zones normales et, a l'inverse, la fluorescence extrinsèque est plus importante sur les zones malades que sur les zones normales.

De manière a rendre visible cette difference, ce document propose de comparer la fluorescence observée dans une bande de fréquence particulière avec la fluorescence totale qui est évaluée indépendamment. Ce document propose ainsi d'acquérir deux images : une image ordinaire et une image de fluorescence en tant qu'entités distinctes et de les comparer afin de voir la difference dans es bandes spectrales des fluorescences intrinsèques et extrinsèques. Il s'agit d'un procédé complexe traitant deux images acquises indépendamment.

On trouve également dans la littérature d'autres applications, notamment dans le cas de la détection in vivo des cancers de l'arbre trachéo-bronchique où l'on a constaté que l'autofluorescence des bronches se modifie lorsque le tissu passe d'un état dysplasique à un état carcinomateux. Dans ce cas, il a été constaté que les lésions entrainaient une diminution de fluorescence dans le vert aux alentours de 500 nm et une augmentation dans la bande de spectre du rouge aux alentours de 600nm.

Ce même principe est aussi utilisé en ophtalmologie pour évaluer le degré de transparence du cristallin dont les protéines photo-oxydées peuvent être mises en évidence par fluorescence.

De telles applications font appel à des dispositifs utilisant des moyens optiques conventionnels utilisant des filtres de séparation spectrale. En particulier, le document US 6 393 315 décrit un appareil pour détecter et repérer des zones enflammées de tissu vivant en utilisant la fluorescence de ces tissus. Une image de fluorescence est obtenue en utilisant trois images de fluorescence acquises chacune pour une de trois bandes spectrales correspondant aux bandes de fluorescence des différents types de tissus. Ensuite, Il est propose de normaliser les signaux de fluorescence dans les trois bandes spectrales entre eux ou avec des signaux reçus dans d'autres bandes spectrales de manière a faire apparaitre ces signaux ensemble avec un maximum de contraste de couleur sur l'image finale. Dans le but d'obtenir une telle image combinée, un ensemble de filtres passe-bande adapté a la selection des fluorescences des chromophores concernés est utilisé.

De tels filtres ont pour inconvénient de nécessiter des dispositifs coûteux, encombrants et fragiles. L'intensité de la lumière doit par ailleurs être importante, ce qui peut entraîner des émissions de fluorescence parasites susceptibles de détériorer le rapport signal/bruit et de masquer la détection du signal pertinent.

La présente invention a pour but de proposer un procédé et un dispositif permettant d'assurer la détection, la localisation, et la caractérisation de différences de structure, ou autres, d'un tissu biologique, ce dispositif étant de constitution simple, d'un faible coût, facile à mettre en oeuvre, et en mesure d'éliminer les différents artéfacts liés aux divers aléas susceptibles d'agir sur la surface du tissu et susceptibles de perturber la mesure.

La présente invention a ainsi pour objet un procédé de détection et de localisation de la différence de densité et/ou de structure et/ou de composition chimique d'un tissu biologique que l'on soumet à un éclairement continu dans une première bande de fréquences déterminée, apte à amener celui-ci à générer un phénomène de fluorescence, d'autofluorescence, ou de luminescence dans une seconde bande de fréquences, **caractérisé en ce qu**'il comporte les étapes consistant :
- à effectuer une saisie d'image du tissu biologique ainsi éclairé, par des moyens vidéo couleur pourvus de capteurs d'images avec une mosaïque de pixels pourvus de filtres de couleurs complémentaires,
- pour chaque point de l'image ainsi obtenue :
   a) recueillir une information en relation avec l'énergie reçue par chaque pixel, de façon à reconstituer l'image du tissu biologique,
   b) amplifier le signal correspondant à l'énergie reçue dans la seconde bande de fréquences de façon à caractériser, ou faire apparaître sur l'image obtenue, ladite différence du tissu biologique.

Suivant l'invention on pourra effectuer un traitement des informations recueillies dans la seconde bande de fréquences, de façon à caractériser la différence de structure obtenue dans une couleur autre que celle correspondant naturellement à cette seconde zone de fréquences.

La présente invention a également pour objet un dispositif de détection et de localisation de la différence de densité et/ou de structure et/ou de composition chimique d'un tissu biologique, **caractérisé en ce qu**'il comprend :
- des moyens aptes à éclairer en continu le tissu biologique avec une lumière se situant dans une première bande de fréquences déterminée, de façon à amener celui-ci à générer un phénomène de fluorescence dans une seconde bande de fréquences,
- des moyens vidéo couleur pourvus de capteurs d'images avec une mosaïque de pixels pourvus de filtres de couleurs complémentaires,
- des moyens de saisie et de calculs aptes, pour chaque point de l'image ainsi obtenue, à recueillir une information en relation avec l'énergie reçue par chaque pixel, de façon à reconstituer l'image du tissu biologique,
- des moyens d'amplification du signal correspondant à l'énergie reçue dans la seconde bande de fréquences, de façon à caractériser, ou faire apparaître sur l'image obtenue, ladite différence du tissu biologique.

Ce dispositif pourra également comprendre des moyens de traitement des informations recueillies dans la seconde bande de fréquences, de façon à caractériser la différence de structure obtenue dans une couleur autre que celle correspondant naturellement à cette seconde zone de fréquences.

La présente invention est particulièrement intéressante en ce que, contrairement aux dispositifs de l'état antérieur de la technique, elle ne fait pas appel à une source de lumière monochromatique, ce qui lui permet d'une part d'utiliser une plus grande partie de l'énergie fournie par la source lumineuse et, d'autre part, en utilisant une bande de radiations se situant dans le domaine du visible, de fournir une image des tissus étudiés (dans le domaine dentaire une image de la dent ou, dans d'autres domaines, l'image d'une muqueuse, de la peau, d'un oeil etc...).

Dans un mode de mise en oeuvre de l'invention, et dans le cas par exemple de l'observation d'un tissu dur tel que l'émail d'une dent, dans lequel la seconde bande de fréquences est centrée sur une couleur fondamentale (dans l'exemple mentionné le rouge), les capteurs CCD des moyens vidéo couleur seront pourvus, au niveau de chacun des pixels, de filtres dont la couleur sera préférentiellement celle des couleurs complémentaires, à savoir le jaune, le magenta, et le cyan. L'utilisation de tels filtres complémentaires est intéressante en ce que d'une part le domaine de réaction de ces filtres, et donc la sensibilité des capteurs, est plus large que celles des couleurs fondamentales et, d'autre part, il est ainsi possible d'agir sur deux signaux, à savoir ceux des couleurs complémentaires associées à chacune des couleurs fondamentales au lieu de ne pouvoir agir que sur un seul signal, à savoir celui associé aux couleurs fondamentales, ce qui permet d'assurer une meilleure gestion des filtrages réalisés.

On décrira ci-après, à titre d'exemple non limitatif, une forme d'exécution de la présente invention, en référence au dessin annexé sur lequel :
La figure 1 est une vue schématique d'un appareil de détection de localisation et de caractérisation de la différence de structure d'un tissu biologique suivant l'invention.
La figure 2 est une vue schématique montrant les domaines des longueurs d'onde auxquels il est fait appel dans le cas d'une application clinique du domaine dentaire suivant la présente invention.

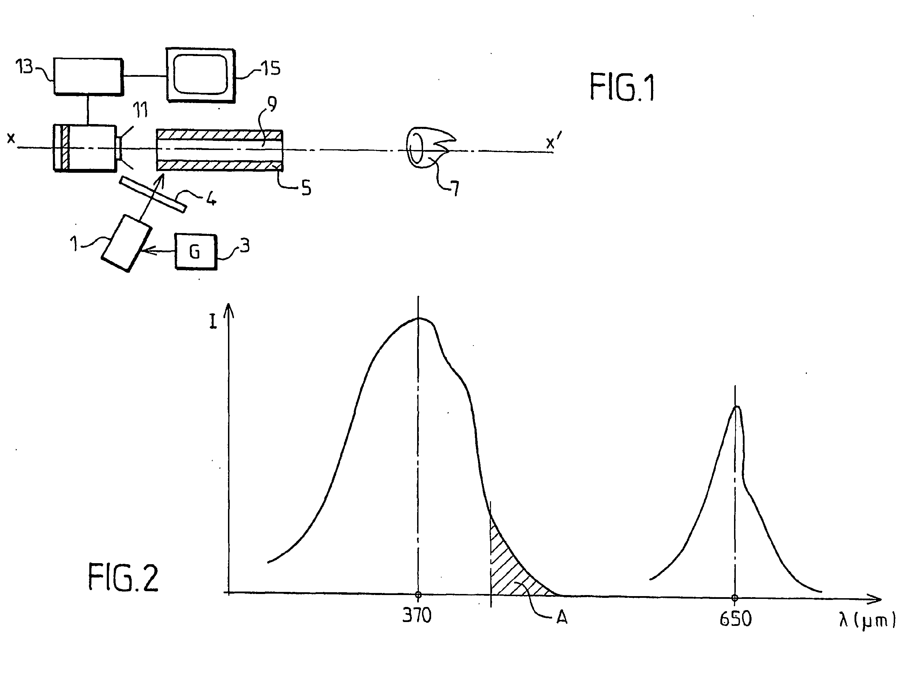

Le dispositif suivant l'invention qui est représenté sur la figure 1 est constitué d'une lampe au xénon 1 qui est alimentée par un générateur de courant 3. La lumière qui est non monochromatique émise par la lampe 1 est filtrée en sortie de lampe par un filtre 4 permettant de conserver en sortie une bande de radiations s'étendant de l'ultraviolet jusqu'au visible proche. Ces radiations lumineuses traversent un tube guide d'ondes 5 et éclairent en continu un tissu biologique, ici constitué par une dent 7 d'un patient. Le tube guide d'ondes 5 est traversé par un canal central et longitudinal d'axe xx' au travers duquel une caméra de prise de vues vidéo couleur 11 est en mesure de filmer la dent 7.

La caméra 11 est reliée à des moyens de traitement des signaux 13 eux-mêmes reliés à des moyens d'affichage vidéo 15.

Le filtre 4 est, dans le présent mode de mise en oeuvre, apte à laisser passer une bande de longueurs d'onde centrée autour de 370nm, une partie de cette bande de fréquences, ainsi que représenté sur la figure 2, comportant une partie A située dans le domaine visible.

On sait que sous l'effet de cet éclairement le constituant minéral de la dent, à savoir l'émail de celle-ci, produit un rayonnement de fluorescence situé dans le domaine du vert et du bleu.

On a, par ailleurs constaté que les parties de l'émail de la dent qui ont subi un début d'altération partielle du fait d'une carie, émettent une radiation de fluorescence dans le domaine des 650 nm, autrement dit au niveau des radiations rouges.

Suivant l'invention, on enregistre à l'aide de la caméra vidéo couleur 11 une image qui est la résultante de plusieurs bandes spectrales à savoir :
- une image de la dent résultant de l'éclairement produit sur celle-ci par la partie visible du spectre d'éclairement,
- une image de la dent provenant de la fluorescence de l'émail de celle-ci générée par son éclairement dans le domaine de l'ultraviolet produit par le spectre d'éclairement,
- une image de fluorescence (dans le domaine du rouge soit environ 650 nm) émise par les zones altérées de l'émail de la dent résultant d'une carie.

Suivant l'invention on réalise une amplification du signal de fluorescence généré dans le rouge (aux environs de 650 nm) par les parties altérées de la dent. Pour ce faire, les pixels des capteurs CCD sont équipés préférentiellement de filtres de couleurs complémentaires, à savoir jaune, magenta et cyan auxquels on adjoint un filtre vert. On comprend dans ces conditions qu'un pixel pourvu par exemple d'un filtre jaune laissera passer les radiations rouges et les radiations vertes si un tel pixel reçoit une énergie lumineuse. Dès lors que ce pixel recevra une énergie lumineuse il conviendra alors de déterminer si celle-ci est une radiation rouge, auquel cas il conviendra de l'amplifier, ou au contraire s'il s'agit d'une radiation verte, auquel cas elle ne sera pas amplifiée. Pour ce faire, on consulte le pixel voisin comportant, lui, un filtre vert et si celui-ci est saturé, cela impliquera que la radiation est verte en totalité et qu'en conséquence il n'y a pas de radiation rouge à amplifier pour ce pixel. Dans le cas inverse il s'agira de rouge ce qui entraînera une amplification.

On procédera ainsi de proche en proche pour l'ensemble des pixels du capteur CCD. Un tel mode opératoire se traduira par l'affichage sur l'écran vidéo 15 d'une part de l'image de la dent (provenant, comme exposé précédemment, d'une part de son éclairement en lumière visible et d'autre part de la fluorescence de l'émail produite dans le domaine des longueurs d'onde bleu/vert), et d'autre part, et superposée à celles-ci, de l'image, de couleur rouge de la carie détectée.

Il serait possible bien entendu suivant l'invention, si besoin en était, de transformer ultérieurement la radiation rouge détectée en des radiations d'affichage de toute autre couleur plus appropriée.

La présente invention permet également, afin de faciliter la détection de la zone altérée de la dent, d'éliminer de l'affichage des fluorescences parasites de couleurs voisines provoquées par d'autres paramètres tels que notamment le tartre, la plaque dentaire, ou des amalgames résultats de traitements antérieurs, ou tout autre élément biologique utile pour le diagnostic recherché.

Il a ainsi été constaté expérimentalement qu'en ajoutant au spectre d'éclairement des radiations situées dans un domaine de longueur d'onde de l'ordre de 400nm on modifiait le spectre de fluorescence produit en décalant la bande de fluorescence des fluorescences parasites.

On pourrait bien entendu, au moyen d'une modification du spectre d'émission, éliminer d'autres phénomènes de fluorescence parasites susceptibles de perturber la mesure et qui seraient dus à la présence sur l'émail de tartre, de plaque dentaire.

On pourrait également suivant l'invention faire appel à des capteurs d'image monochromes, notamment de type CCD. Les moyens de saisie d'images seraient alors constitués d'une part d'un premier capteur de luminance et d'autre part d'un capteur pourvu d'un filtre de la couleur correspondant à celle de la fluorescence émise lors de la détection de la différence recherchée. Par exemple dans le cas de recherche d'une carie ce filtre aura une couleur laissant passer une radiation de 650 nm et dans le cas de la recherche de tissus dysplasiques ou carcinomateux il aura une couleur laissant passer une radiation de 500 nm. Bien entendu le dispositif suivant l'invention ne pourra alors que détecter des anomalies d'un seul type. Il serait bien entendu possible de prévoir alors d'autres capteurs monochromes équipés d'autres filtres permettant chacun d'accéder à une application supplémentaire.

Dans un mode de réalisation particulier de l'invention, la caméra 11 pourra être pourvue de moyens lui permettant de fonctionner soit en détection de fluorescence, soit en visualisation de la zone observée en image vidéo conventionnelle. Pour cela, l'objectif sera pourvu d'un filtre correspondant à une atténuation de la lumière émise. La pièce à main de la caméra sera pourvue d'un commutateur permettant d'utiliser le filtre dédié quand on est en fluorescence ou de le désactiver pour l'imagerie vidéo conventionnelle. En mode fluorescence, il sera par ailleurs possible de mettre un filtre couleur devant l'objectif pour en améliorer le contraste.

Bien que la mise en oeuvre de la présente invention ait été principalement décrite en regard d'applications se situant principalement dans le domaine dentaire, on pourra également utiliser celle-ci pour la détection et la localisation d'altérations tissulaires telles que celles de la muqueuse bronchique dont l'autofluorescence dans le vert (500nm environ) diminue et celle dans le rouge augmente aux alentours des 600nm.

De même on pourrait faire appel à des capteurs autres que CCD et notamment à des capteurs CMOS.

On pourra également détecter et localiser des lésions de tissus, tels que ceux du pancréas, qui éclairés dans une bande de fréquences centrée sur une radiation de longueur d'onde 400 nm, génèrent une augmentation significative de la fluorescence dans le rouge (630nm).

## Revendications

1. Procédé de détection et de localisation de la différence de densité et/ou de structure et/ou de composition chimique d'un tissu biologique (7) que l'on soumet à un éclairement continu dans une première bande de fréquences déterminée, apte à amener celui-ci à générer un phénomène de fluorescence, d'autofluorescence, ou de luminescence dans une seconde bande de fréquences, **caractérisé en ce qu'**il comporte les étapes consistant:
- à effectuer une saisie d'image du tissu biologique ainsi éclairé, par des moyens vidéo couleur pourvus de capteurs d'images avec une mosaïque de pixels pourvus de filtres de couleurs complémentaires, ces filtres ayant un domaine de réaction plus large que celui des couleurs fondamentales,
- pour chaque point de l'image ainsi obtenue:
a) recueillir une information en relation avec l'énergie reçue par chaque pixel, de façon à reconstituer l'image du tissu biologique (7),
b) amplifier le signal correspondant à l'énergie reçue dans la seconde bande de fréquences de façon à caractériser, ou faire apparaître sur l'image obtenue, ladite différence du tissu biologique (7), cette amplification du signal correspondant à l'énergie reçue dans la seconde bande de fréquences étant réalisée de proche en proche pour l'ensemble des pixels du capteur d'image en agissant sur les deux signaux des couleurs complémentaires associées à chacune des couleurs fondamentales.

2. Procédé selon la revendication 1, **caractérisé en ce que** les couleurs complémentaires sont le cyan, le magenta et le jaune.

3. Procédé selon la revendication 2, **caractérisé en ce que** un filtre vert est adjoint aux filtres de couleurs complémentaires.

4. Procédé selon la revendication 3, **caractérisé en ce que**, l'émail d'une dent ayant subi un début d'altération du fait d'une carie émettant une radiation de fluorescence dans le domaine des 650 nm, autrement dit au niveau des radiations rouges, on réalise une amplification du signal de fluorescence généré aux environs de 650 nm par les parties altérées de la dent, cette amplification étant réalisée de proche en proche pour l'ensemble des pixels du capteur CCD à la manière suivante :
- si un pixel jaune reçoit une énergie lumineuse, on consulte le pixel voisin comportant, lui, un filtre vert,
- si celui-ci est saturé, cela impliquera que la radiation est verte en totalité et qu'il n'y a pas de radiation rouge à amplifier pour ce pixel jaune qui n'est donc pas amplifié,
- sinon l'énergie lumineuse reçue sur le pixel jaune correspond à une radiation rouge, auquel cas il est amplifié.

5. Procédé selon la revendication 1, **caractérisé en ce que** l'on on effectue un traitement des informations recueillies dans la seconde bande de fréquences, de façon à caractériser la différence de structure obtenue dans une couleur autre que celle correspondant naturellement à cette seconde zone de fréquences.

6. Procédé suivant l'une des revendications 1 ou 2, **caractérisé en ce que** l'on ajoute à la bande de fréquences du spectre d'éclairement des radiations aptes à modifier le spectre de fluorescence pour décaler la bande de fluorescence des fluorescences parasites.

7. Dispositif de détection et de localisation de la différence de densité et/ou de structure et/ou de composition chimique d'un tissu biologique (7), **caractérisé en ce qu'**il comprend:
- des moyens (1) aptes à éclairer en continu le tissu biologique (7) avec une lumière se situant dans une première bande de fréquences déterminée, de façon à amener celui-ci à générer un phénomène de fluorescence dans une seconde bande de fréquences,
- des moyens vidéo couleur (11) pourvus de capteurs d'images avec une mosaïque de pixels pourvus de filtres de couleurs complémentaires, ces filtres ayant un domaine de réaction plus large que celui des couleurs fondamentales,
- des moyens de saisie et de calculs aptes, pour chaque point de l'image ainsi obtenue, à recueillir une information en relation avec l'énergie reçue par chaque pixel de façon à reconstituer l'image du tissu biologique (7),
- des moyens d'amplification du signal correspondant à l'énergie reçue dans la seconde bande de fréquences de façon à caractériser, ou faire apparaître sur l'image obtenue, ladite différence du tissu biologique, cette amplification du signal correspondant à l'énergie reçue dans la seconde bande de fréquences étant réalisée de proche en proche pour l'ensemble des pixels du capteur d'image en agissant sur les deux signaux des couleurs complémentaires associées à chacune des couleurs fondamentales.

8. Dispositif suivant la revendication 7, **caractérisé en ce qu'**il comprend des moyens de traitement (13) des informations recueillies dans la seconde bande de fréquences, de façon à caractériser la différence de structure obtenue dans une couleur autre que celle correspondant naturellement à cette seconde zone de fréquences.

## Claims

1. Method for detecting and locating the difference in density and/or structure and/or chemical composition of a biological tissue (7) which is subjected to continuous illumination in a first determined band of frequencies, able to cause the tissue to generate a phenomenon of fluorescence, auto-fluorescence or luminescence in a second band of frequencies, **characterized in that** it comprises the steps consisting of:
capturing an image of the biological tissue illuminated in this way, using colour video means provided with image sensors with a mosaic of pixels provided with filters of complementary colours, these filters having a range of reaction greater than those of the primary colours,
for each point of the image so obtained:
a) collecting data related to the energy received by each pixel, so as to reconstitute the image of the biological tissue (7),
b) amplifying the signal corresponding to the energy received in the second band of frequencies so as to characterize or cause to appear the said difference of the biological tissue (7) in the image obtained, said amplification of the signal corresponding to the energy received in the second band of frequency being realized from pixel to pixel for all the pixels of the sensor by acting on the two signals of complementary colours associated with each of the primary colours.

2. Method as in claim 1, **characterized in that** said complementary colours are cyan, magenta and yellow.

3. Method as claimed in claim 2, **characterized in that** a green filter is added to filters of complementary colours.

4. Method as claimed in claim 3, **characterized in that**, the enamel of a tooth being subject of deterioration caused by decay emitting a fluorescence radiation around 650 nm, i.e. in red radiations, the amplification of the fluorescence signal generated around 650 nm by deteriorated parts of the tooth is realized from pixel to pixel for all the pixels of the sensor in the following way:
- if a yellow pixel receives light energy, the neighbouring green pixel is consulted;
- if this last green pixel is saturated, this implies that the radiation is entirely green and that therefore there is no red radiation to be amplified for this yellow pixel that is thus not amplified;
- in the reverse case, the light energy received by the yellow pixel corresponds to a red radiation and is thus amplified.

5. Method as claimed in claim 1, **characterized in that** the data collected in the second band of frequencies is processed so as to characterize the structure difference obtained in a colour other than the colour naturally corresponding to this second zone of frequencies.

6. Method as claimed in claim 1 or 2, **characterized in that** radiations are added to the band of frequencies of the illumination spectrum that are able to modify the fluorescence spectrum to shift the fluorescence band of parasite fluorescence.

7. Device for detecting and locating the difference in density and/or structure and/or chemical composition of a biological tissue (7), **characterized in that** it comprises:
means (1) able to illuminate the biological tissue (7) continuously with a light located in a first determined band of frequencies, so as to cause the tissue to generate a phenomenon of fluorescence in a second band of frequencies,
colour video means (11) provided with image sensors with a mosaic of pixels provided with filters of complementary colours, these filters having a range of reaction greater than those of the primary colours,
capture and calculation means which, for each point of the image so obtained, are able to collect data related to the energy received by each pixel so as to reconstitute the image of the biological tissue (7),
means for amplifying the signal corresponding to the energy received in the second band of frequencies so as to characterize or cause to appear the said difference of the biological tissue in the image obtained, said amplification means of the signal corresponding to the energy received in the second band of frequency being such that said amplification is realized from pixel to pixel for all the pixels of the sensor by acting on the two signals of complementary colours associated with each of the primary colours.

8. Device as claimed in claim 7, **characterized in that** it comprises processing means (13) to process data collected in the second band of frequencies, so as to characterize the structure difference obtained in a colour other than the colour naturally corresponding to this second zone of frequencies.

## Patentansprüche

1. Verfahren zum Feststellen und zum Lokalisieren eines Unterschieds in der Dichte und/oder in der Struktur und/oder in der chemischen Zusammensetzung eines biologischen Gewebes (7), das einer kontinuierlichen Belichtung in einem ersten bestimmten Frequenzband unterzogen wird, die in der Lage ist, dieses dazu zu bringen, ein Fluoreszenz-, Autofluoreszenz- oder Lumineszenzphänomen in einem zweiten Frequenzband zu erzeugen, **dadurch gekennzeichnet, daß** es die Schritte umfaßt, die darin bestehen:
- eine Bildaufnahme des so belichteten biologischen Gewebes mit Farbvideomitteln durchzuführen, die mit Bildsensoren mit einem Mosaik aus Pixeln ausgestattet sind, die mit Komplementärfarbfiltern ausgestattet sind, wobei diese Filter einen größeren Reaktionsbereich als den der Grundfarben aufweisen,
- für jeden so erhaltenen Bildpunkt:
a) eine Information bezogen auf die von jedem Pixel empfangene Energie zu sammeln, um das Bild des biologischen Gewebes (7) zu rekonstituieren,
b) das Signal zu verstärken, das der Energie entspricht, die in dem zweiten Frequenzband empfangen wird, um den Unterschied des biologischen Gewebes (7) zu charakterisieren oder auf dem erhaltenen Bild erscheinen zu lassen, wobei diese Verstärkung des Signals, das der in dem zweiten Frequenzband empfangenen Energie entspricht, nach und nach für die Gesamtheit der Pixel des Bildsensors ausgeführt wird, indem auf die beiden Signale der Komplementärfarben, die mit jeder der Grundfarben assoziiert sind, eingewirkt wird.

2. Verfahren nach Anspruch 1, **dadurch gekennzeichnet, daß** die Komplementärfarben Cyan, Magenta und Gelb sind.

3. Verfahren nach Anspruch 2, **dadurch gekennzeichnet, daß** den Komplementärfarbfiltern ein Grünfilter hinzugefügt ist.

4. Verfahren nach Anspruch 3, **dadurch gekennzeichnet, daß**, wenn ein Zahnschmelz, der eine beginnende Veränderung aufgrund von Karies erfahren hat, eine Fluoreszenzstrahlung im Bereich von 650 nm, anders gesagt im Bereich der Rotstrahlungen, ausstrahlt, eine Verstärkung des Fluoreszenzsignals, das bei etwa 650 nm durch die veränderten Stellen des Zahns erzeugt wird, ausgeführt wird, wobei diese Verstärkung nach und nach für die Gesamtheit der Pixel des CCD-Sensors auf folgende Weise ausgeführt wird:
- wenn ein gelber Pixel eine Lichtenergie empfängt, wird der benachbarte Pixel konsultiert, der seinerseits einen Grünfilter enthält,
- wenn dieses gesättigt ist, würde das implizieren, daß die Strahlung vollständig grün ist und daß es keine zu verstärkende Rotstrahlung für das gelbe Pixel gibt, die daher nicht verstärkt wird,
- andernfalls entspricht die Lichtenergie, die auf dem gelben Pixel empfangen wird, einer Rotstrahlung, die in diesem Fall verstärkt wird.

5. Verfahren nach Anspruch 1, **dadurch gekennzeichnet, daß** eine Behandlung der in dem zweiten Frequenzband gesammelten Informationen durchgeführt wird, um den Strukturunterschied zu charakterisieren, der in einer Farbe erhalten wurde, die anders ist als diejenige, die diesem zweiten Frequenzbereich natürlich entspricht.

6. Verfahren gemäß einem der Ansprüche 1 oder 2, **dadurch gekennzeichnet, daß** dem Frequenzband des Belichtungsspektrums Strahlungen zugegeben werden, die in der Lage sind, das Fluoreszenzspektrum zu modifizieren, um das Fluoreszenzband der Störfluoreszenzen zu verschieben.

7. Vorrichtung zum Feststellen und zum Lokalisieren eines Unterschieds in der Dichte und/oder in der Struktur und/oder in der chemischen Zusammensetzung eines biologischen Gewebes (7), **dadurch gekennzeichnet, daß** sie umfaßt:
- Mittel (1), die in der Lage sind, das biologische Gewebe (7) mit einem Licht, das in einem ersten bestimmten Frequenzband liegt, kontinuierlich zu belichten, um dieses dazu zu bringen, ein Fluoreszenzphänomen in einem zweiten Frequenzband zu erzeugen,
- Farbvideomittel (11), die mit Bildsensoren mit einem Mosaik aus Pixeln ausgestattet sind, die mit Komplementärfarbfiltern ausgestattet sind, wobei diese Filter einen größeren Reaktionsbereich als den der Grundfarben aufweisen,
- Aufnahme- und Berechnungsmittel, die in der Lage sind, für jeden so erhaltenen Bildpunkt eine Information bezogen auf die Energie zu sammeln, die von jedem Pixel empfangen wird, um das Bild des biologischen Gewebes (7) zu rekonstituieren,
- Mittel zur Verstärkung des Signals, das der Energie entspricht, die in dem zweiten Frequenzband empfangen wird, um die Differenz des biologischen Gewebes zu charakterisieren oder auf dem erhaltenen Bild erscheinen zu lassen, wobei diese Verstärkung des Signals, das der in dem zweiten Frequenzband empfangenen Energie entspricht, nach und nach für die Gesamtheit der Pixel des Bildsensors ausgeführt wird, indem auf die beiden Signale der Komplementärfarben, die mit jeder der Grundfarben assoziiert sind, eingewirkt wird.

8. Vorrichtung gemäß Anspruch 7, **dadurch gekennzeichnet, daß** sie Mittel zur Behandlung (13) der in dem zweiten Frequenzband gesammelten Informationen umfaßt, um die Strukturdifferenz zu charakterisieren, die in einer Farbe erhalten wurde, die anders ist als diejenige, die diesem zweiten Frequenzbereich natürlich entspricht.
